# EUROPEAN PATENT SPECIFICATION

(11) **EP 3 769 680 B1**
(45) Date of publication and mention of the grant of the patent: **10.04.2024**
(21) Application number: 19770630.2
(22) Date of filing: 28.01.2019
(51) Int. Cl.: A61B 5/1473, A61B 5/145, A61B 5/00, A61B 5/1486

(54) **SENSOR INSERTION DEVICE**
VORRICHTUNG ZUM EINSETZEN EINES SENSORS
DISPOSITIF D'INSERTION DE CAPTEUR

(30) Priority: 23.03.2018 JP 2018056500; 23.03.2018 JP 2018056582; 29.06.2018 JP 2018124530
(43) Date of publication of application: 27.01.2021
(73) Proprietor: PHC Holdings Corporation, Tokyo 105-8433 (JP)
(72) Inventor: SHIMIZU, Ryuji, Ehime 791-0395 (JP); NISHIO, Akira, Ehime 791-0395 (JP); KOUGE, Masahiro, Ehime 791-0395 (JP)
(74) Representative: Eisenführ Speiser
(86) International application number: PCT/JP2019/002656
(87) International publication number: WO 2019/181199

(56) References cited:
- WO-A1-2011/026130
- WO-A1-2016/012482
- WO-A1-2017/070360
- CN-U- 206 777 330
- JP-A- 2005 511 191
- JP-A- 2013 523 217
- JP-A- 2015 531 660
- US-A- 5 304 193
- US-A1- 2018 064 376

## Description

### TECHNICAL FIELD

The present invention relates to a sensor insertion device that inserts a sensor for measuring biological information into a patient's body to perform continuous blood glucose measurement, for example.

### BACKGROUND ART

The configuration of a conventional sensor insertion device is as follows.

Specifically, a conventional sensor insertion device comprises a lower case that has an upper surface opening, an upper case that covers the outer periphery of the lower case from above, a sensor base that is disposed inside the lower case and holds a sensor, a needle holder that is disposed above the sensor base so as to be movable up and down and in which a guide needle of the sensor is held, and a needle holder raising mechanism that raises the needle holder.

The needle holder raising mechanism is disposed inside the upper case, and includes a spring that is compressed when the upper case is pushed down. After the needle holder has been lowered, the stored energy in this spring is released to raise the needle holder (for example, Patent Literature 1).

Document W 2017/070360 A1 proposes systems and methods for measuring an analyte in a host, more particularly, sensor applicators and methods of use with activation that implant the sensor, withdraw the insertion needle, engage the transmitter with the housing, and disengage the applicator from the housing which allow for such steps to occur without significant loss of spring force, and without deleterious effects such as seal slingshotting.

Document WO 2016/012482 A1 proposes an insertion device for inserting an analyte sensor into a body tissue, wherein the insertion device comprises an insertion needle holder and a drive mechanism for linearly driving the insertion needle holder in a longitudinal direction. The drive mechanism comprises at least one actuator for actuating the drive mechanism. The actuator comprises at least one actuator arm, which is pivotable about at least one axle in order to actuate the drive mechanism. The insertion device further comprises at least one protection against reuse including at least one locking mechanism. The locking mechanism is adapted to at least partially prevent a back-pivoting of the actuator arm, in a direction reversing the actuation direction once the actuator arm has been pivoted by at least one threshold angle.

### CITATION LIST

### PATENT LITERATURE

Patent Literature 1: Japanese Unexamined Patent Application Publication No.2015-509011

### SUMMARY

The invention is solely defined by the appended claims.

### TECHNICAL PROBLEM

A problem encountered with the conventional example given above is that when the guide needle is pulled out from the upper arm of the patient, the patient ends up experiencing more discomfort.

That is, with a conventional sensor insertion device, the guide needle is pulled out from the upper arm of the patient by the release of the stored energy of the spring, and the guide needle that has been pulled out hits a stopper and comes to a stop. The loud snap produced when the guide needle and the stopper collide increases discomfort (fear) on the part of the patient.

In view of this, it is an object of the present invention to reduce patient discomfort when using a sensor insertion device.

### SOLUTION TO PROBLEM

In order to achieve this object, the sensor insertion device of the present disclosure comprises a lower case that has an upper surface opening; an upper case that is disposed around the outer periphery of the lower case, covering from above, and slidably with respect to the lower case, and that is operated downward by a user when performing a needle insertion operation; a sensor base that is disposed inside the lower case and that holds a sensor for acquiring biological information; a needle holder that is disposed above the sensor base so as to be movable up and down, and that holds a guide needle which is to be inserted into a patient's body in order to guide the sensor into the body; and a needle holder raising mechanism that raises the needle holder holding the guide needle that has been inserted into the patient's body, wherein the needle holder raising mechanism has a pinion gear that raises the needle holder; a first rack gear that is provided to the needle holder and is engaged with the pinion gear; and a second rack gear that is provided to the upper case and is engaged with the pinion gear. As the upper case descends, the pinion gear raises the needle holder by engagement with the second rack gear.

The present invention achieves its intended purpose by the configuration of claim 1.

### ADVANTAGEOUS EFFECTS

As described above, with the present invention, after the guide needle of the needle holder has been inserted, the needle holder is raised by the pinion gear provided to the needle holder raising mechanism, so there is no snap noise produced by a spring, and patient discomfort (fear) can be reduced.

### BRIEF DESCRIPTION OF DRAWINGS

FIG. 1 is an oblique view of the usage state of the sensor insertion device according to Embodiment 1;
FIG. 2 is a front view of the mounted state of the sensor base and the biological information measuring device in FIG. 1;
FIG. 3 is an oblique view of the sensor insertion device in FIG. 1;
FIG. 4 is an exploded oblique view of the sensor insertion device in FIG. 1;
FIG. 5 is an exploded oblique view of the sensor insertion device in FIG. 1;
FIG. 6 is an exploded perspective view of the lower case of the sensor insertion device in FIG. 1;
FIG. 7 is an exploded cross section of the sensor insertion device in FIG. 1;
FIG. 8 is a partially cut-away oblique view of the lower case of the sensor insertion device in FIG. 1;
FIG. 9 is an exploded oblique view of the upper case of the sensor insertion device in FIG. 1;
FIG. 10 is a cross section of the sensor insertion device in FIG. 1;
FIG. 11 is a cross section of the sensor insertion device in FIG. 1;
FIG. 12 is a detail cross section of FIG. 11;
FIG. 13 is a detail cut-away oblique view of the upper case of the sensor insertion device in FIG. 1;
FIG. 14 is a cross section of the operating state of the sensor insertion device in FIG. 1;
FIG. 15 is a detail cross section of FIG. 14;
FIG. 16 is a cross section of the operating state of the sensor insertion device in FIG. 1;
FIG. 17 is a detail cross section FIG. 16;
FIG. 18 is a cross section of the operating state of the sensor insertion device in FIG. 1;
FIG. 19 is a cross section of the operating state of the sensor insertion device in FIG. 1;
FIG. 20 is a cross section of the operating state of the sensor insertion device in FIG. 1;
FIG. 21 is a detail oblique view of FIG. 20;
FIG. 22 is an oblique view of the usage state of the sensor insertion device according to Embodiment 2;
FIG. 23 is a front view of the usage state of the sensor base and the biological information measuring device in FIG. 22;
FIG. 24 is an oblique view of the sensor insertion device in FIG. 22;
FIG. 25 is an exploded oblique view of the sensor insertion device in FIG. 22;
FIG. 26 is a partially cut-away oblique view of the sensor insertion device in FIG. 22;
FIG. 27 is a partially cut-away oblique view of the sensor insertion device in FIG. 22;
FIG. 28 is a detail oblique view of the A portion of the sensor insertion device in FIG. 26;
FIG. 29 is a detail oblique view of the B portion of the sensor insertion device in FIG. 26;
FIG. 30 is a detail oblique view of the C portion of the sensor insertion device in FIG. 26;
FIG. 31 is a cross section of the operating state of the sensor insertion device in FIG. 22;
FIG. 32 is a cross section of the operating state of the sensor insertion device in FIG. 22;
FIG. 33 is a cross section of the operating state of the sensor insertion device in FIG. 22;
FIG. 34 is a cross section of the operating state of the sensor insertion device in FIG. 22;
FIG. 35 is a partially cut-away oblique view of a partial modification example of the sensor insertion device in FIG. 26; and
FIG. 36 is a detail oblique view of the D portion of the sensor insertion device in FIG. 35.

### DESCRIPTION OF EMBODIMENTS

Embodiments of the present disclosure will now be described in detail with reference to the drawings.

### Embodiment 1

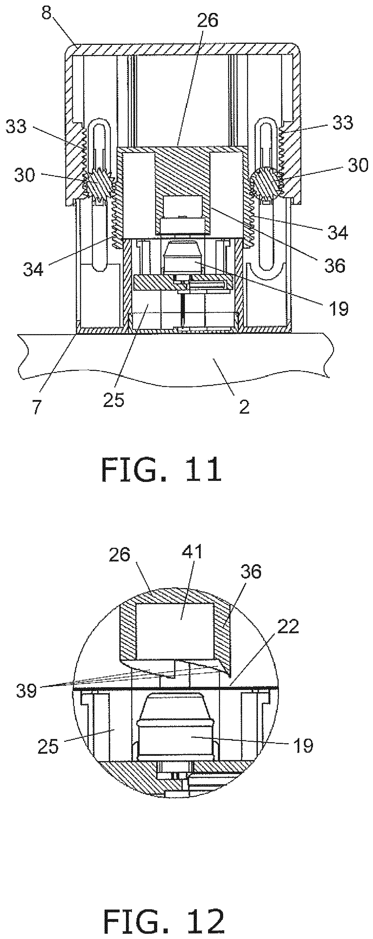

In Embodiment 1, "upper" and "lower" refer to "upper" and "lower" in the usage state of the sensor insertion device 1 shown in FIG. 11, etc. (a state in which the sensor is attached to a patient 2). The "up and down direction" means the insertion direction of the guide needle 20 in the sensor insertion device 1 (the needle insertion direction and the needle extraction direction).

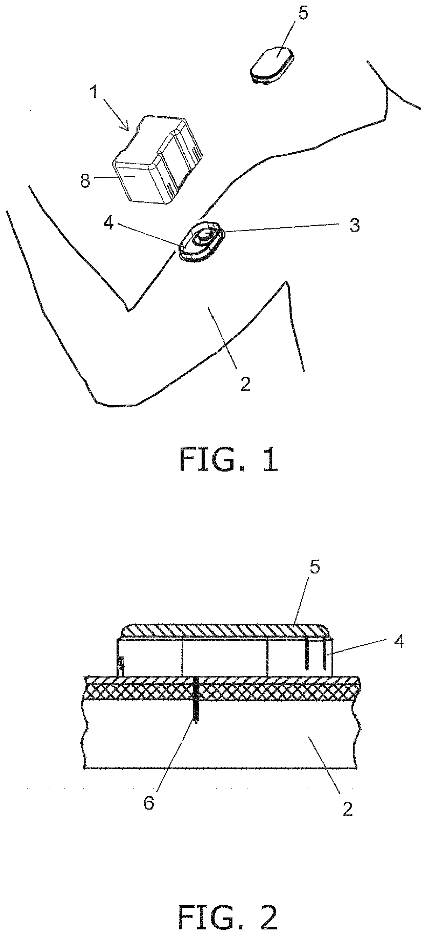

FIG. 1 shows the usage state of the sensor insertion device 1. In FIG.1, a sensor unit 3 is mounted on a part of the body of the patient 2 along with a sensor base 4 by means of the sensor insertion device 1. After this, a measuring device 5 (an example of a biological information measuring device) is mounted on the upper surface of the sensor base 4.

FIG. 2 shows a state in which the sensor base 4 and the measuring device 5 are mounted on the patient 2. In FIG. 2, a sensor 6 of the sensor unit 3 is left in the body of the patient 2. Consequently, biological information about the patient 2 is sensed by the sensor unit 3 and measured by the measuring device 5. The measurement result is transmitted from the measuring device 5 to a mobile telephone (not shown), for example.

In this embodiment, the biological information to be measured is the blood glucose level. For example, so-called continuous blood glucose measurement is performed in which a patient's blood glucose level is continuously measured every five minutes for one or two weeks. This makes it possible to spot any tendencies of the glucose level of the patient within a certain period, and to ascertain the glucose level during sleep.

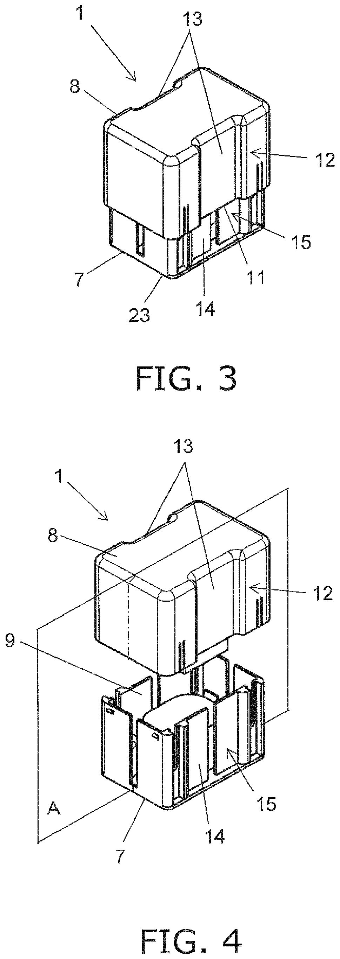

As shown in FIGS. 3 to 10, the sensor insertion device 1 in this embodiment comprises a lower case 7, an upper case 8, a sensor base 4, a needle holder 26, and a needle holder raising mechanism 30A.

The lower case 7 and the upper case 8 have a rectangular shape that is wider than it is long in plan view.

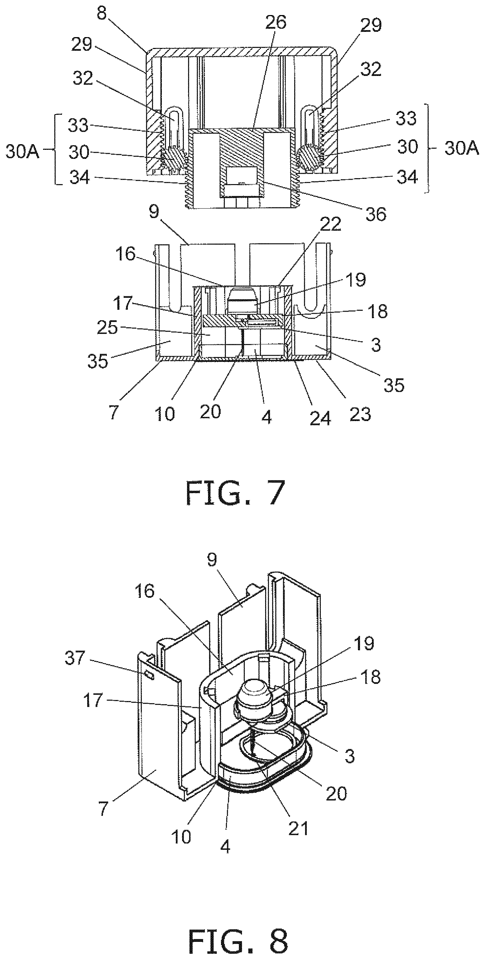

As shown in FIG. 4, the lower case 7 has an upper surface opening 9 formed on the upper surface side, and as shown in FIGS. 7 and 8, has a bottom outlet 10 formed on the bottom side in order to eject the sensor unit 3 and the sensor base 4 downward.

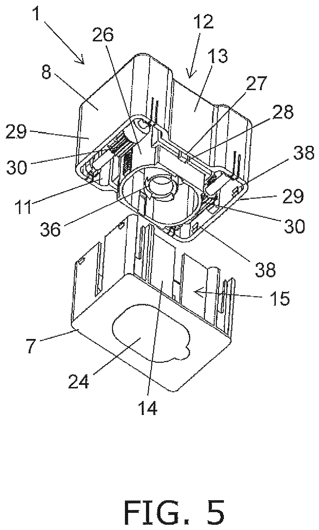

As shown in FIG. 5, the upper case 8 has a lower surface opening 11, and as shown in FIG. 3, covers the outer periphery of the lower case 7 from above. More specifically, the upper case 8 and the lower case 7 both have a rectangular shape that is wider than it is long in plan view, and are stacked one over the other so that the lower case 7 is disposed on the inside.

As shown in FIG. 3, grip portions 13 are provided to the two long side surfaces 12 of the upper case 8 disposed opposite each other, extending from top to bottom in the center portion in the longitudinal direction, and recessed in a stepped shape from the outer surface side to the inner surface side. Also, slide guides 14, which protrude in a stepped shape from the outer surface side to the inner surface side of the grip portions 13 and over which inward protrusions slide, are provided as shown in FIGS. 4 to 6 to the portions of the lower case 7 corresponding to the grip portions 13, to line up with the grip portions 13.

The slide guides 14 are provided to the two long side surfaces 15 of the lower case 7 disposed opposite each other, and are formed so as to protrude in a stepped shape from the inner surface, in the central portion in the longitudinal direction, from top to bottom. In a state in which the rear surfaces of the grip portions 13 of the upper case 8 are in contact with the surface of the slide guides 14, these rear surfaces are slid in the up and down direction (usage state).

Because the long side surfaces 12 of the upper case 8 are thus provided with the grip portions 13 that are recessed toward the inner surface side, the grip portions 13 are smaller, and do not protrude out from the outer surface of the upper case 8. Therefore, as shown in FIG. 1, it is easier for user to hold the upper case 8 in the right hand. Also, because the grip portions 13 are provided in the central portion of the long side surfaces 12 of the upper case 8, they afford a more stable grip, so the usage state (discussed below) is extremely stable.

The user of the sensor insertion device 1 may be the patient himself, or may be a third party such as a nurse or a caregiver.

The detailed configurations of the various components will be described below.

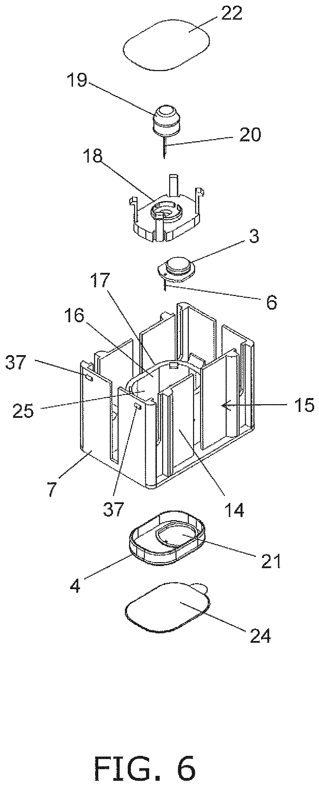

As shown in FIGS. 6 to 8, a cylindrical antibacterial wall 17 is provided in the lower case 7. The antibacterial wall 17 has an upper opening 16 and a bottom outlet 10, and is provided so as to protrude from around the periphery of the bottom outlet 10 toward the inside of the lower case 7, so as to surround the bottom outlet 10.

As mentioned above, the sensor base 4 is disposed at the bottom outlet 10 portion in the interior of the antibacterial wall 17. The antibacterial wall 17 has in its interior a needle unit 19 held by a sensor holder 18, above the sensor base 4. More precisely, as shown in FIG. 8, the sensor holder 18 holds the needle unit 19 on the upper surface side, and holds the above-mentioned sensor unit 3 on the lower surface side.

In this state, the sensor unit 3 of the sensor 6 (see FIG. 6) is being held inside the guide needle 20 of the needle unit 19. When the guide needle 20 is lowered by a needle insertion operation, the sensor 6 is inserted into the body of the patient 2 along with the guide needle 20, after which just the guide needle 20 is pulled upward and withdrawn. Consequently, just the sensor 6 is left in the body of the patient 2 (see FIG. 2).

The sensor base 4 has formed in it a through-hole 21 for moving the guide needle 20 up and down in a state of being inserted.

One of the main features of this embodiment is that, as shown in FIG. 8, the sensor unit 3, the sensor holder 18, the sensor base 4, the needle unit 19, and the guide needle 20 are housed within the antibacterial wall within 17. In this state, as shown in FIG. 7, an antibacterial sheet (first antibacterial sheet) 22 that is sealed so as to cover the upper opening 16 is attached to the upper opening 16 of the antibacterial wall 17.

Also, as shown in FIG. 7, an antibacterial sheet (second antibacterial sheet) 24 that seals the bottom outlet 10 so as to cover the bottom outlet 10 is mounted on the bottom surface 23 of the lower case 7 (see FIG. 5). The antibacterial sheet 24 has a larger surface area than the opening portion of the bottom outlet 10, and is bonded to the bottom surface 23. Thus, the antibacterial sheet 24 constitutes a part of the bottom surface 23 of the lower case 7.

That is, an antibacterial chamber 25 has the upper opening 16 on the upper side, and the bottom outlet 10 (an example of the lower opening of the antibacterial chamber 25) on the lower side. The upper opening 16 is sealed by the antibacterial sheet 22, and the bottom outlet 10 is sealed by the antibacterial sheet 24. Consequently, the space inside the antibacterial wall 17 becomes the antibacterial chamber 25.

In the state prior to the use of the sensor insertion device 1, the sensor unit 3, the sensor holder 18, the sensor base 4, the needle unit 19, the guide needle 20, and the like are housed inside the antibacterial chamber 25 as mentioned above.

In a state in which the space inside the antibacterial wall 17 has been sealed by the antibacterial sheet 22 and the antibacterial sheet 24, it is irradiated from the outside with an electron beam. Consequently, the sensor unit 3, the sensor holder 18, the sensor base 4, the needle unit 19, and the guide needle 20 disposed within the antibacterial wall 17 are subjected to a sterilization treatment, and are kept in a sterile state.

Meanwhile, as shown in FIG. 5, the cylindrical needle holder 26 whose the lower surface side is open is disposed inside the upper case 8. The needle holder 26 is provided so as to be movable by a needle holder raising mechanism 30A (discussed below; see FIG. 7) within the sensor insertion device 1 in the up and down direction. Furthermore, the upper case 8 is provided with engaging arms 27 in between the needle holder 26 and the grip portions 13.

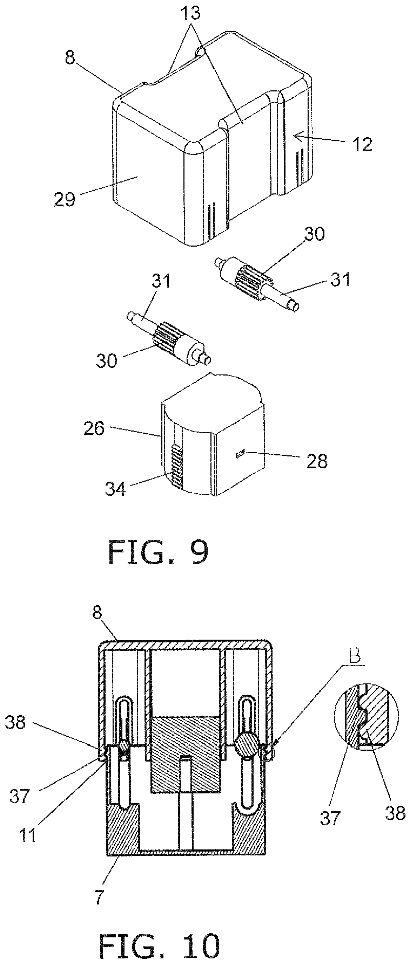

The engaging arms 27 are engaged with engagement holes 28 (see FIG. 9) formed in the side surfaces of the needle holder 26. During needle insertion, the user pushes down on and lowers the upper case 8, whereupon the engagement between the engaging arms 27 and the engagement holes 28 causes the needle holder 26 to descend along with the upper case 8. At this point, the pinion gear 30 is lowered along with the needle holder 26 and the upper case 8 in a non-rotating state.

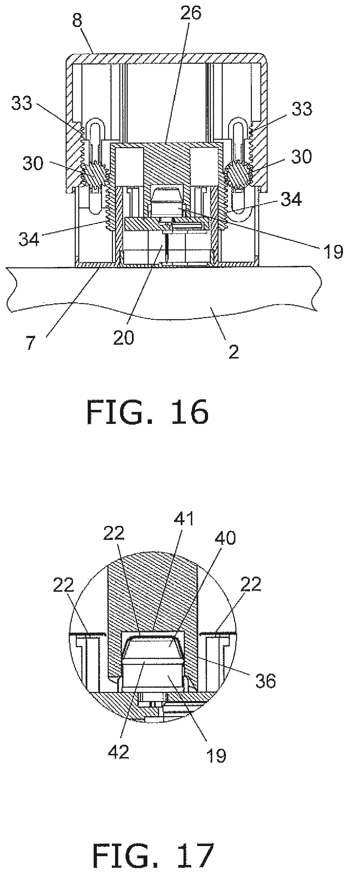

As will be described in detail below, during descent of the upper case 8, needle holder 26 is holding the needle unit 19 (see FIG. 16), and the result of the needle holder 26 descending through the sensor insertion device is that the needle insertion operation of the guide needle 20 is performed. Also, in the needle removal operation following the needle insertion operation, the guide needle 20 is pulled out when the needle holder 26 rises inside the sensor insertion device 1.

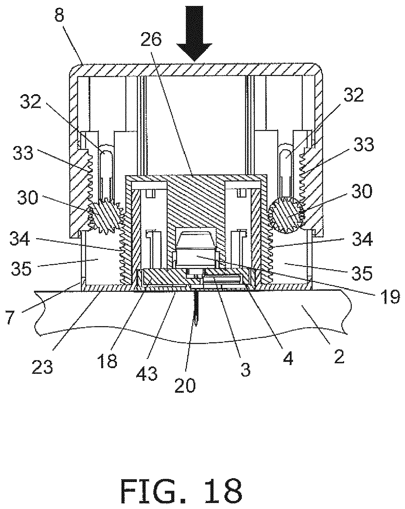

The sensor insertion device 1 of this embodiment is provided with the needle holder raising mechanism 30A that raises the needle holder 26 in order to pull the guide needle 20 (see FIG. 18) that was inserted into the body of the patient 2 out of the body.

More specifically, as shown in FIG. 7, etc., the needle holder raising mechanism 30A has two pinion gears 30 disposed so as to sandwich the needle holder 26, and rack gears 33 and 34 that engage with the pinion gear 30.

As shown in FIG. 5, the two pinion gears 30 that raise the needle holder 26 are disposed between the needle holder 26 in upper case 8 and the short side surfaces 29 of the upper case 8.

Also, as shown in FIG. 7, shaft support grooves 32 that support the shafts 31 of the pinion gears 30 (see FIG. 9) in a state of being movable in the up and down direction are formed along the up and down direction of the upper case 8 on the inside of the long side surfaces 12 of the upper case 8. The pinion gears 30 are provided so as to be slidable in the up and down direction along the shaft support grooves 32 during the sensor mounting shown in FIG. 11, etc.

Rack gears (first rack gears) 33 that engage with the pinion gears 30 are provided along the up and down direction on the inner surface side of the upper case 8. The rack gears 33 are disposed at the portions opposite the pinion gears 30 on the inner surface side of the short side surfaces 29. The lower portions of the rack gears 33 are engaged with the two pinion gears 30.

Rack gears (second rack gears) 34 are provided on the outer surface side of the needle holder 26, along the up and down direction on both side surfaces opposite the pinion gears 30. The two pinion gears 30 are engaged with the upper portions of the rack gears 34 at both side surfaces of the needle holder 26.

As shown in FIG. 7, etc., the rack gears 33 and 34 are disposed such that their engagement teeth are opposite each other.

The two pinion gears 30 are disposed so as to be sandwiched on both sides by the rack gears 33 and 34, and do not rotate until the needle insertion operation shown in FIG. 18 is complete. Once the needle insertion operation shown in FIG. 18 is complete, the user then pushes down on the upper case 8, whereupon the rack gears 33 descend relative to the needle holder 26, rotating the pinion gears 30, on both side surfaces of the needle holder 26. When the pinion gears 30 rotate, the rack gears 34 engaged with the pinion gears 30 are driven by the pinion gears 30, causing the needle holder 26 to slide upward. As a result, the needle holder 26 is lifted up (raised) by the pinion gears 30 on both sides, and the guide needle 20 held by the needle holder 26 is pulled out of the body of the patient 2.

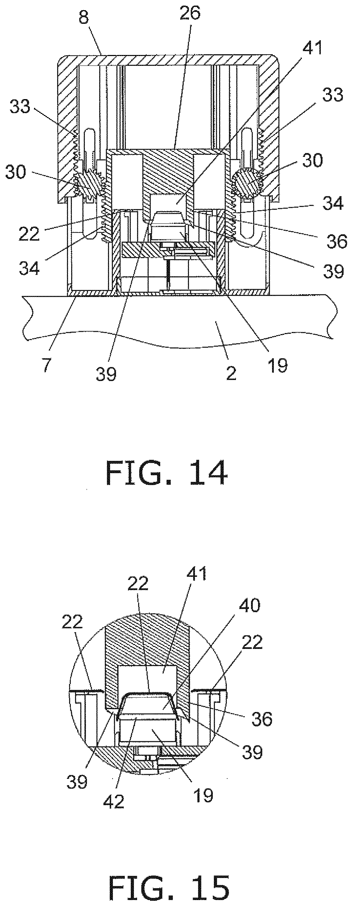

That is, the pinion gears 30, the rack gears 33, and the rack gears 34 constitute the needle holder raising mechanism 30A that raises the needle holder 26. With this configuration, after the needle insertion operation shown in FIG. 14, the upper case 8 is further operated downward (the direction of needle insertion into the patient 2), whereupon the needle holder 26 can be slid upward inside the upper case 8.

Accordingly, the user can perform the needle insertion operation and the needle extraction operation with the guide needle 78 using the sensor insertion device 1 by the same operation as when pushing down on the upper case 8 (in the direction of inserting the needle into the patient 2). This makes the device more convenient for the user.

Also, the needle holder 26 is provided with a needle carrier 36 whose lower end side is cylindrical, as shown in FIG. 7. The needle carrier 36 is disposed opposite the needle unit 19 that is disposed in the lower case 7. When the lower end side of the needle carrier 36 is engaged with the needle unit 19, the needle holder 26 holds the guide needle 20 of the needle unit 19.

As described above, because the grip portions 13, which are recessed inward, are provided to the long side surfaces 12 of the upper case 8, the grip portions 13 are smaller, so that it is easier for the user to grasp the grip portions 13. In this embodiment, the pinion gears 30 are disposed in the upper case 8 at the positions of the long side surfaces 12 of the upper case 8 where the grip portions 13 are not formed, that is, at both side portions of the grip portions 13 on the long side surfaces 12. Thus, pinion gears 30 are provided at positions away from the recessed part of the grip portions 13 of the upper case 8. Accordingly, the grip portions 13 can be favorably recessed inward, allowing the upper case 8 to have a compact shape that is easy to hold.

Engagement protrusions 37 (see FIG. 6) are provided in the four corners on the upper end side of the short sides of the lower case 7. Also, concave portions 38 are provided in the four corners on the lower end side of the short sides of the upper case 8.

As discussed above, when the upper case 8 is placed over the lower case 7, the four engagement protrusions 37 (see FIG. 6) of the lower case 7 engage with the four concave portions 38 of the upper case 8 as shown in FIG. 10.

When using the sensor insertion device 1 configured as above, the user lifts up the sensor insertion device 1 by grasping the grip portions 13 of the upper case 8 shown in FIG. 3, peels off the antibacterial sheet 24 (see FIG. 5) from the bottom surface 23 of the lower case 7, and then presses the bottom surface 23 of the lower case 7 against his or her body 2.

FIG. 11 is a cross section of the sensor insertion device 1 pressed against the patient's body 2, and is a cross section along the A plane in FIG. 4. FIG. 12 and FIGS. 14 to 20 are also cross sections along the A plane in FIG. 4.

FIG. 12 is a detail cross section of the area around the needle holder 26 in FIG. 11. Inside the upper case 8, the needle holder 26 is provided at a portion opposite the upper surface of the antibacterial sheet 22. The needle holder 26 is provided with the needle carrier 36 that engages with and holds the needle unit 19 in the antibacterial chamber 25 when the upper case 8 is lowered.

The lower end side of the needle carrier 36 is disposed opposite the upper end side of the needle unit 19, with the antibacterial sheet 22 in between.

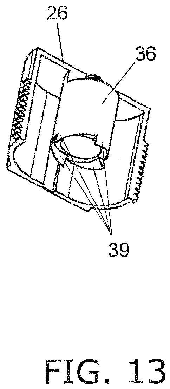

Furthermore, the lower end portion of the needle carrier 36 has a cylindrical shape which holds an outer peripheral surface of the needle unit 19 as shown in FIG. 13. Four blades 39 are formed on a lower opening edge of the cylindrical form of the needle carrier 36, and the blades 39 are provided to cut out the antibacterial sheet 22.

When the user pushes down on the upper case 8 with at least a specific amount of force from the state shown in FIG. 11 to a state in which the grip portions 13 are grasped, the engagement between the engagement protrusions 37 of the lower case 7 and the concave portions 38 of the upper case 8 (see FIG. 10) is released, and the upper case 8 is pushed downward all at once. At this point, the grip portions 13 of the upper case 8 are guided by the slide guides 14 of the lower case 7 while descending (see FIG. 3).

As shown in FIG. 9, the engaging arms 27 of the upper case 8 are engaged with the engagement holes 28 of the needle holder 26 on the inner surface side of the grip portions 13. Therefore, the needle holder 26 descends along with the upper case 8.

With the needle carrier 36 of the needle holder 26, as shown in FIGS. 14 and 15, the distal end portions of the four blades 39 provided at the lower end poke through and break the antibacterial sheet 22.

That is, in this embodiment, the antibacterial chamber 25 is provided inside the lower case 7 that is unitized with the upper case 8. Therefore, the sterile state of the antibacterial chamber 25 is maintained until the time of its actual use, so a hygienic state can be maintained.

Also, the needle unit 19 has a conical portion 40 (or a truncated conical portion) protruding upward at its upper end. Accordingly, the antibacterial sheet 22 broken by the blades 39 is pushed into an antibacterial sheet holder 41 provided in the needle carrier 36, by the conical portion 40 of the needle unit 19.

When the upper case 8 is further depressed, as shown in FIGS. 16 and 17, the needle holder 26 holds the outer surface of the needle unit 19 on the inner wall surface of the needle carrier 36 (the inner wall surface of the antibacterial sheet holder 41).

In this embodiment, in a state in which the needle carrier 36 holds the needle unit 19, the top of the conical portion 40 of the needle unit 19 is inserted into the antibacterial sheet holder 41 of the needle carrier 36.

Therefore, the broken antibacterial sheet 22 is pushed into the antibacterial sheet holder 41 by the conical portion 40 of the needle unit 19. In this state, the antibacterial sheet 22 is then pressed against the inner wall surface of the antibacterial sheet holder 41 by an O-ring 42 provided on the side surface of the needle unit 19.

As a result, the broken antibacterial sheet 22 is held inside the antibacterial sheet holder 41, and therefore does not hinder the operation of the sensor insertion device 1.

When the upper case 8 is further depressed in a state in which the needle holder 26 holds the needle unit 19, the sensor unit 3 held by the sensor holder 18 is mounted on the upper surface side of the sensor base 4 as shown in FIG. 18.

At this point, the sensor 6 protrudes downward from the bottom surface 23 of the lower case 7 along with the guide needle 20, and the skin is punctured by the guide needle 20. The sensor base 4 is attached to the patient's body 2 by an adhesive portion 43 provided on the lower surface of the sensor base 4. That is, the sensor 6 is inserted into the body 2 along with the guide needle 20.

When the guide needle 20 is inserted, the shafts 31 of the pinion gears 30 move downward in the shaft support grooves 32 and until reaching shaft supports 35 provided to the lower case 7.

The operation of extracting the guide needle 20 will now be described.

In this embodiment, as shown in FIG. 7, the pinion gears 30, the rack gears 33, and the rack gears 34 constitute the needle holder raising mechanism 30A that raises the needle holder 26. In the operation of removing the guide needle 20, the pinion gears 30 are rotated by engagement with the rack gears 33 and the rack gears 34 as the upper case 8 descends further upon completion of the needle insertion operation, and the needle holder 26 is raised. As a result, the guide needle 20 is extracted from the patient's body 2.

This will be described in detail below.

When the user further depresses the upper case 8 after the needle insertion operation shown in FIG. 18, the pinion gears 30 are supported by the shaft supports 35 as shown in FIG. 18, and therefore do not go down any further.

On the other hand, when the upper case 8 slides further downward, the rack gears 33 inside the upper case 8 slide downward. Therefore, the pinion gears 30 engaged with the rack gears 33 on the upper case 8 side rotate on both sides of the needle holder 26. This rotational force is transmitted to the rack gears 34 on the lower case 7 side that are engaged with the pinion gears 30.

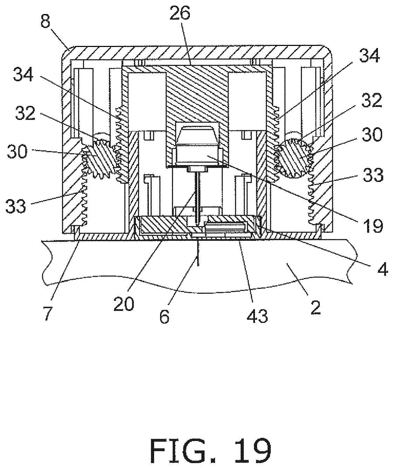

Therefore, the needle holder 26 is lifted up by receiving an upward drive force from the rack gears 34 disposed on both sides, and slides upward (rises) from the state shown in FIG. 18 to the state shown in FIG. 19.

As a result, the guide needle 20 held by the needle holder 26 is extracted from the patient's body 2 and stowed in the lower case 7 as shown in FIG. 19. The sensor 6 is left in the patient's body 2.

The pinion gears 30 rotate while sliding in the upper case 8 along the shaft support grooves 32 with which the shafts 31 on both sides are engaged.

The force by which the pinion gears 30 lift the needle holder 26 is greater than the force of engagement between the engaging arms 27 of the upper case 8 and the engagement holes 28 of the needle holder 26. Therefore, the engagement between the upper case 8 and the needle holder 26 is released, and the needle holder 26 can rise through the upper case 8.

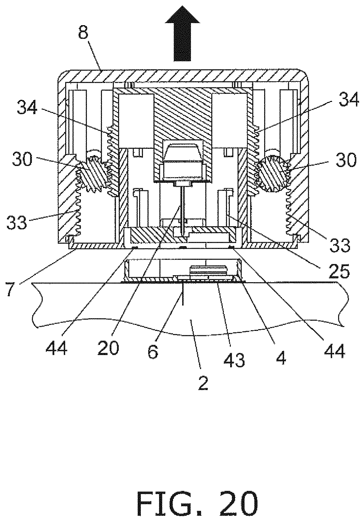

After this, when the user lifts up the upper case 8, as shown in FIG. 20, since the sensor base 4 is affixed to the patient's body 2 by the adhesive portion 43, the sensor base 4 detaches from holding prongs 44 in the antibacterial chamber 25 and comes off.

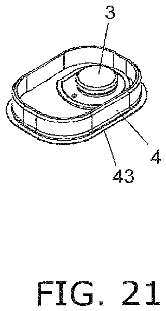

As a result, as shown in FIG. 21, the sensor base 4 is attached to the patient's body 2 in a state in which the sensor unit 3 is being held, and this concludes the series of operations.

As described above, the sensor insertion device 1 of this embodiment comprises the lower case 7, the upper case 8, the sensor base 4, the needle holder 26, and the needle holder raising mechanism 30A. The lower case 7 has the upper surface opening 9. The upper case 8 is placed over the outer periphery of the lower case 7 and is disposed slidably with respect to the lower case, and is operated downward by the user when performing a needle insertion operation. The sensor base 4 is disposed in the lower case 7 and holds the sensor 6. The needle holder raising mechanism 30A raises the needle holder 26, which is disposed so as to be movable up and down above the sensor base 4 and holds the guide needle 20 of the sensor 6. The needle holder raising mechanism 30A has the pinion gears 30, the rack gears 34, and the rack gears 33. The pinion gears 30 raise the needle holder 26. The rack gears 34 are provided to the needle holder 26 and engage with the pinion gears 30. The rack gears 33 are provided to the upper case 8 and engage with the pinion gears 30. As the upper case 8 descends, the pinion gears 30 raise the needle holder 26 by engagement with the rack gears 33.

Therefore, after the guide needle 20 of the needle holder 26 has been inserted, the needle holder 26 can be raised by the pinion gears 30 provided in the upper case 8 by further lowering the upper case 8. As a result, no snapping sound is generated by a spring, and patient discomfort (fear) can be reduced.

Also, the sensor insertion device 1 of this embodiment comprises the lower case 7, the upper case 8, the antibacterial chamber 25, the antibacterial sheet 22, the antibacterial sheet 24, and the needle carrier 36. The lower case 7 has the upper surface opening 9 and the bottom outlet 10. The upper case 8 is placed over the outer periphery of the lower case 7 from above and is disposed slidably with respect to the lower case 7, and is operated downward by the user when performing a needle insertion operation. The antibacterial chamber 25 is provided in the lower case 7 and houses the needle unit 19, which includes the guide needle 20 to be inserted into the patient's body 2, and the sensor unit 3, which includes the sensor 6 for acquiring a blood glucose level. The antibacterial sheet 22 is attached to the upper opening of the antibacterial chamber 25. The antibacterial sheet 24 is attached to the bottom outlet 10 corresponding to the lower opening of the antibacterial chamber 25. The needle carrier 36 is provided to the portion in the upper case 8 that is opposite the upper surface of the antibacterial sheet 22, and engages with the needle unit 19 in the antibacterial chamber 25 when the upper case 8 is lowered.

That is, since the antibacterial chamber is provided in the lower case 7 that is unitized with the upper case 8, the sterile state of the antibacterial chamber is maintained until the time of its actual use, and as a result, a hygienic state can be maintained.

### Embodiment 2

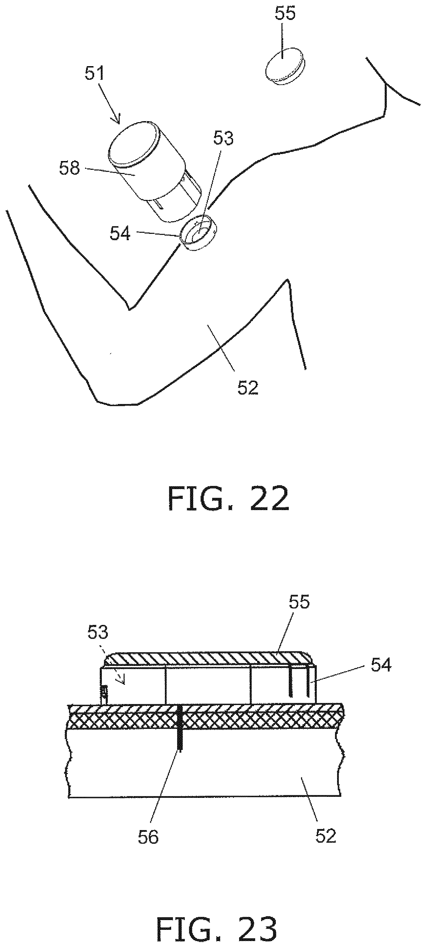

As shown in FIG. 22, the sensor insertion device 51 according to this embodiment is used to attach a disc-shaped sensor base 54 holding a sensor unit 53 to a patient's body 52 (upper arm, etc.).

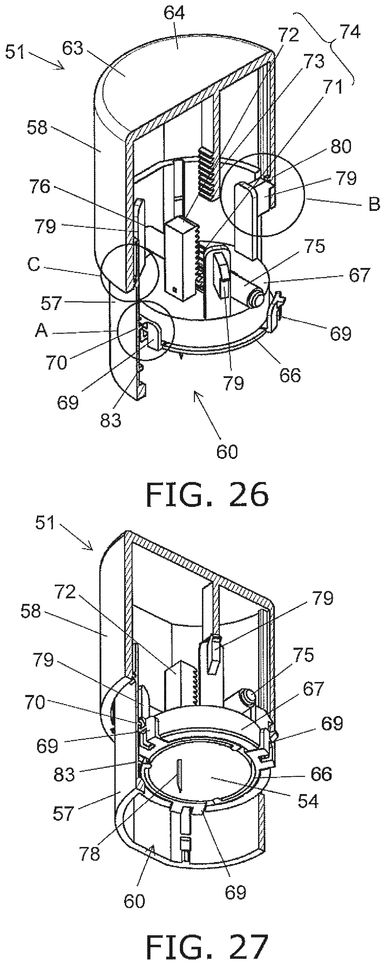

As shown in FIG. 26, just as with the sensor insertion device 1 in Embodiment 1 above, the sensor insertion device 51 constitutes a needle holder raising mechanism 74 that raises a needle holder 67 by using a pinion gear 71 and two rack gears (a first rack gear 72 and a second rack gear 73). Since the needle holder 67 is raised by the pinion gear 71 included in the needle holder raising mechanism 74, no snapping sound is generated by a spring, and patient discomfort (fear) can be reduced.

This will now be described in detail with reference to the drawings.

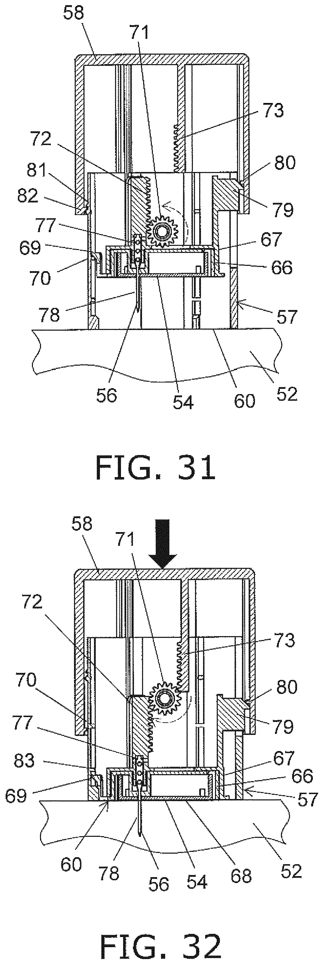

In Embodiment 2, "upper" and "lower" refer to "upper" and "lower" in the usage state of the sensor insertion device 51 shown in FIG. 31, etc. (a state in which the sensor is attached to a patient 52). The "up and down direction" means the insertion direction of a guide needle 78 in the sensor insertion device 51 (the needle insertion direction and the needle extraction direction).

FIG. 22 is a diagram showing the usage state of the sensor insertion device 51. In FIG. 22, with the sensor insertion device 51, a sensor unit 53 is attached to a part of the patient's body (such as an upper arm) 52 along with a sensor base 54. After this, a measuring device 55 (an example of a biological information measuring device; see FIG. 23) is mounted on the upper surface of the sensor base 54.

FIG. 23 shows a state in which the sensor base 54 and the measuring device 55 have been attached to the patient's body (upper arm) 52. In FIG. 23, a sensor 56 included in the sensor unit 53 is left in the patient's body 52. Consequently, biological information about the patient's body 52 is sensed by the sensor 56 and measured by the measuring device 55. The measurement result is transmitted from the measuring device 55 to a mobile telephone (not shown), for example.

In this embodiment, the biological information to be measured is the blood glucose level. For example, so-called continuous blood glucose measurement is performed in which a patient's blood glucose level is continuously measured every five minutes for one or two weeks. This makes it possible to spot any tendencies of the glucose level of the patient within a certain period, and to ascertain the glucose level during sleep.

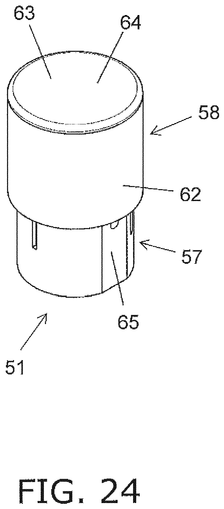

As shown in FIGS. 24 to 27, the sensor insertion device 51 in this embodiment comprises a lower case 57, an upper case 58, a sensor base 54, a needle holder 67, and a needle holder raising mechanism 74.

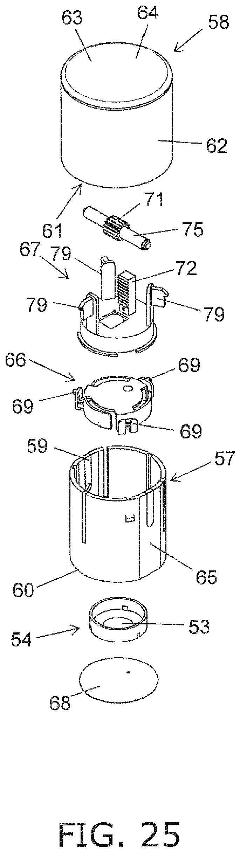

As shown in FIG. 25, the lower case 57 has a cylindrical shape, an upper surface opening 59 is provided on the upper surface side of the cylindrical shape, and a bottom outlet 60 is provided on the lower surface side for ejecting the sensor base 54 downward.

As shown in FIG. 25, the sensor base 54 is a substantially cylindrical member and holds in its interior the sensor unit 53 including the sensor 56 shown in FIG. 23.

As shown in FIG. 25, the upper case 58 has a cylindrical shape that is closed on the upper surface side open on the lower surface side, and has a lower surface opening 61. The upper case 58 is placed from above so as to cover the outer peripheral surface of the lower case 57 (see FIG. 24).

That is, the upper case 58 has a cylindrical shape like the lower case 57, and these are put together so as to be stacked one above the other. The outer peripheral surface of the upper case 58 is provided with grip portions 62 that are gripped by the user. Furthermore, a contact portion 64 that makes contact with the user's palm during use is provided on the upper surface portion 63 of the upper case 58. Also, cylindrical slide guides 65 are provided on the outer peripheral surface of the lower case 57 at the portions corresponding to the grip portions 62 of the upper case 58.

As a result, during use of the sensor insertion device 51, the user can use the five fingers of the left hand to grasp the grip portions 62 of the upper case in a state in which the palm of the right hand is in contact with the contact portion 64. This makes the usage state (discussed below) extremely stable.

The user of the sensor insertion device 51 may be the patient himself, or may be a third party such as a nurse or a caregiver.

The detailed configurations of the various components will be described below.

As shown in FIG. 25, the sensor base 54 holding the sensor 56 (see FIG. 23), a base holder 66 holding the sensor base 54, and a needle holder 67 placed over the base holder 66 are disposed in the lower case 57. A skin tape 68 for attaching the sensor base 54 to the patient's body 52 is provided to the lower surface of the sensor base 54.

FIGS. 26 and 27 show the sensor insertion device 51 when not in use (before sensor attachment).

As shown in FIG. 27, the sensor base 54 is inserted from the lower side of the base holder 66. FIG. 27 shows a state in which the skin tape 68 has been removed, in order to illustrate the state of the sensor base 54 and the base holder 66.

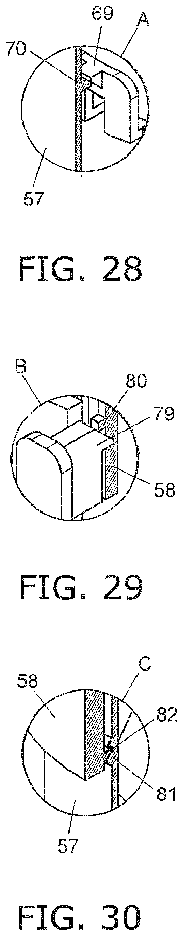

The base holder 66 is such that three base engaging portions 69 provided at substantially equal angular intervals along the outer peripheral portion engage with first engaging prongs 70 provided at substantially equal angular intervals in the middle of the lower case 57 (see A in FIG. 26, and FIG. 28). The needle holder 67 is placed over the outer peripheral surface of the base holder 66. The needle holder 67, the base holder 66, and the sensor base 54 are held in the middle of the lower case 57, and slide downward from this middle position when attached to the patient.

The needle holder 67 is substantially disk shaped, and is provided movably in the lower case 57 in the up and down direction (the insertion direction). The pinion gear 71, the first rack gear 72, and the second rack gear 73 constituting the needle holder raising mechanism 74 for raising the needle holder 67 are disposed on the upper surface side of the needle holder 67, as shown in FIG. 26.

The needle holder raising mechanism 74 is disposed near the center of the lower case 57 and the needle holder 67. More precisely, the pinion gear 71 is provided near the center axis of the cylindrical lower case 57 and the substantially disk-shaped needle holder 67.

The pinion gear 71 is connected to a shaft 75 extending from the left and right ends of the gear portion.

The shaft 75 is provided as the rotational axis of the pinion gear 71, and is disposed in a state in which its longitudinal direction is perpendicular to the central axis of the lower case 57. The ends of the shaft 75 are supported by shaft supports 76 provided on the inner surface of the lower case 57. Therefore, the pinion gear 71 rotates in the middle of the lower case 57 while being supported by the shaft supports 76.

The first rack gear 72, which has a substantially quadrangular prism shape, is engaged with the pinion gear 71. The first rack gear 72 is provided so as to protrude upward from the upper surface of the needle holder 67. The first rack gear 72 is engaged with the pinion gear 71 on the lower side of the gear portion in the pre-use state shown in FIG. 27.

Also, as shown in FIG. 31, the upper part of the needle unit 77 is embedded in the interior of the first rack gear 72 on the lower side thereof. The guide needle 78 held by the needle unit 77 protrudes from the lower surface side of the sensor base 54 below the first rack gear 72.

The guide needle 78 includes the sensor 56 for measuring biological information (blood glucose level, etc.).

In the pinion gear 71, as shown in FIG. 26, a second rack gear 73 having a substantially quadrangular prism shape is disposed on the opposite side from the first rack gear 72, at the upper part of the pinion gear 71.

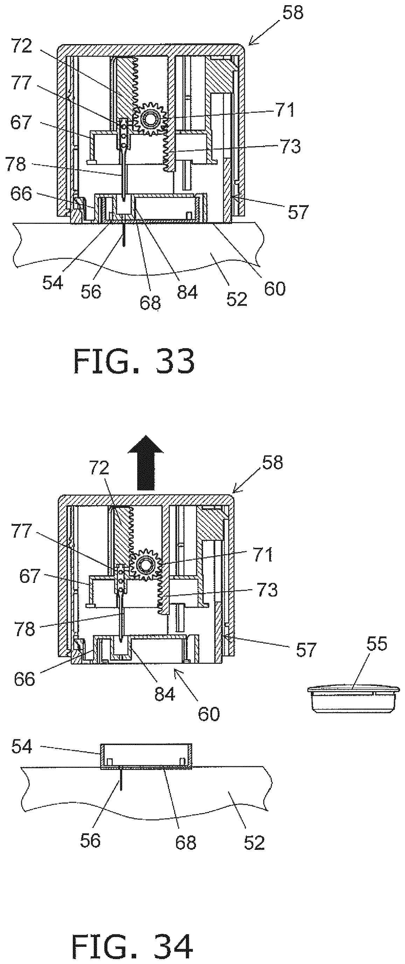

The upper end side of the second rack gear 73 is a fixed end that is fixed to the ceiling of the upper case 58, and the lower end side is a flexible free end. Accordingly, the lower end side of the second rack gear 73 is movable outward in the radial direction from the center axis of the upper case 58. The second rack gear 73 is disposed so as to sandwich the pinion gear 71 from the left and right sides along with the first rack gear 72. That is, the first rack gear 72 and the second rack gear 73 are disposed such that their engaging teeth face each other in a state in which the upper case 58 has been lowered (see FIGS. 32 and 33), and the pinion gear 71 is disposed therebetween. Therefore, the second rack gear 73 engages with the pinion gear 71 on the opposite side from the first rack gear 72.

Thus, the needle holder raising mechanism 74 is disposed near the central axis of the lower case 57 and the needle holder 67. This allows the needle holder 67 that has undergone the needle insertion operation to be pulled up stably. This needle extraction operation will be described below.

The needle holder 67 is provided with three (a plurality of) needle holder engaging portions 79 at equal angular intervals along the outer peripheral portion of its upper surface. Needle holder pressing portions 80 are provided on the inner surface of the upper case 58 at the portions opposite the needle holder engaging portions 79 (see the B portion in FIG. 26, and FIG. 29).

That is, since the needle holder pressing portions 80 are engaged with the needle holder engaging portions 79, when the upper case 58 is lowered, the needle holder 67 is pushed (slid) downward.

Furthermore, as shown in FIG. 31, a lower case engaging portion 81 is provided on the upper part of the outer periphery of the lower case 57. The lower case engaging portion 81 is engaged with an upper case engaging prong 82 provided at the lower part of the inner periphery of the upper case 58 (see the C portion in FIG. 26, and FIG. 30).

When using the sensor insertion device 51 configured as above, the user grasps the upper case 58 and lifts up the sensor insertion device 51, and presses the bottom outlet 60 of the lower case 57 against his body 52. FIGS. 31 to 34 are cross sections of the sensor insertion device 51.

From here, the operation of inserting the guide needle 78 is performed.

More specifically, the user presses the upper case 58 downward (toward his body 52) from the state shown in FIG. 31. At this point, when the upper case 58 is pressed with at least a certain amount of force, the engagement between the lower case engaging portion 81 and the upper case engaging prong 82 (see FIG. 30) is released, and the hold of the upper case 58 on the lower case 57 is released. Accordingly, the upper case 58 is pushed down all at once. As the upper case 58 descends, the grip portions 62 (outer peripheral surface) are guided by the slide guides 65 (outer peripheral surface) of the lower case 57 (see FIG. 24).

At this point, as shown in FIG. 29, the needle holder pressing portions 80 on the upper case 58 press the needle holder engaging portions 79 downward, so that the needle holder 67 descends along with the upper case 58 and pushes the base holder 66 down.

Consequently, in the base holder 66, the three base engaging portions 69 on the outer periphery are disengaged from the first engaging prongs 70, and the hold of the base holder 66 on the lower case 57 is released. Therefore, as the needle holder 67 held by the upper case 58 descends, the base holder 66 and the sensor base 54 descend all at once.

That is, the engagement force between the needle holder pressing portions 80 on the upper case 58 side and the needle holder engaging portions 79 on the needle holder 67 side greater than the engagement force between the base engaging portions 69 on the base holder 66 side and the first engaging prongs 70 on the lower case 57 side.

Consequently, the engagement between the needle holder pressing portions 80 on the upper case 58 side and the needle holder engaging portions 79 on the needle holder 67 side is maintained, while the engagement of the base holder 66 with the lower case 57 is released, allowing the base holder 66 to be lowered.

When the needle holder 67 is lowered, the first rack gear 72 fixed to the upper surface of the needle holder 67 rotates the engaged pinion gear 71 counterclockwise, going from the state shown in FIG. 31 to the state shown in FIG. 32. More specifically, the pinion gear 71 rotates counterclockwise while moving from the lower end to the upper end of the first rack gear 72.

In the state prior to the needle insertion operation shown in FIG. 31, the pinion gear 71 is engaged with the first rack gear 72 and not engaged with the second rack gear 73.

At this point, as shown in FIG. 32, the guide needle 78 protrudes downward from the bottom outlet 60 of the lower case 57 and is inserted into the patient's body 52. The sensor 56 guided by the guide needle 78 is inserted into the patient's body 52 together with the guide needle 78.

At this point, as shown in FIG. 32, the base holder 66 is held by the lower case 57 because the base engaging portions 69 are engaged with second engaging prongs 83 of the lower case 57. The sensor base 54 is attached to the patient's body 52 by the adhesion of the skin tape 68 affixed to the lower surface side.

At this point, as shown in FIG. 32, the first rack gear 72 is engaged with the pinion gear 71 on its upper side. At the pinion gear 71, the lower end side of the second rack gear 73 is lowered and engages on the opposite side from the side engaged with the first rack gear 72.

Specifically, when the needle insertion operation shown in FIG. 32 is complete, the pinion gear 71 is engaged with both the first rack gear 72 and the second rack gear 73, and this concludes the preparation for the needle extraction operation (discussed below).

In this state, the operation of inserting the guide needle 78 ends.

Next, the operation of extracting the guide needle 78 will be described.

In this embodiment, as shown in FIG. 26, the pinion gear 71, the first rack gear 72, and the second rack gear 73 constitute the needle holder raising mechanism 74 that raises the needle holder 67. In the operation of extracting the guide needle 78, the pinion gear 71 is rotated clockwise by engagement with the first rack gear 72 and the second rack gear 73 as the upper case 58 further descends after the completion of the needle insertion operation, and this raises the needle holder 67. As a result, the guide needle 78 is removed from the patient's body 2.

The needle extraction operation will now be described in specific terms.

When the user further presses the upper case 58 down from the state in which the needle insertion operation shown in FIG. 32 is complete, the engagement between the needle holder pressing portions 80 and the needle holder engaging portions 79 is released, and the upper case 58 descends with respect to the needle holder 67 and the lower case 57.

Accordingly, the second rack gear 73 fixed to the upper case 58 moves downward, engages with the pinion gear 71 from the lower end (the free end side), and rotates the pinion gear 71 clockwise. This rotational force is transmitted through the pinion gear 71 to the first rack gear 72. As a result, as shown in FIG. 33, the first rack gear 72 rises. Therefore, the needle holder 67 to which the first rack gear 72 is fixed is pulled up by the clockwise rotation of the pinion gear 71, and slides (rises) upward.

As a result, the guide needle 78 held by the needle holder 67 is pulled out of the patient's body 52 and stowed in a needle accommodation portion 84 provided to the base holder 66, leaving the sensor 56 in the patient's body 52. Therefore, the guide needle 78 protruding from the bottom outlet 60 of the lower case 57 is stowed in the lower case 57. The sensor 56 is left inside the patient's body 52.

After this, when the user lifts up the upper case 58, as shown in FIG. 34, since the sensor base 54 is affixed to the patient's body 52 by the adhesion of the skin tape 68, the sensor base 54 is detached from the base holder 66 and remains on the surface of the patient's body 52. Consequently, the sensor base 54 is ejected from the bottom outlet 60 of the lower case 57 together with the sensor 56.

As a result of the above operation, the sensor base 54 is mounted on the patient's body 52 in a state in which the sensor 56 is inserted into the patient's body 52, as shown in FIG. 34.

This concludes the series of operations (needle insertion operation and needle extraction operation) by the sensor insertion device 51.

Accordingly, the user can perform the needle insertion operation and the needle extraction operation of the guide needle 78 using the sensor insertion device 51 by the same operation, namely, pushing down on the upper case 58 (the direction of inserting the needle into patient's body 52). This makes the device more convenient for the user to use.

At this point, the guide needle 78 extracted from the patient's body 52 is stowed in the needle accommodation portion 84 provided to the base holder 66, so that it is protected by the base holder 66. That is, the base holder 66 becomes a lid that covers the bottom outlet 60, and the guide needle 78 is covered by the base holder 66. This means that the user or another person will not accidentally touch the guide needle 78, which lowers the risk of blood infection.

As described above, with the sensor insertion device 51 in this embodiment, after the guide needle 78 is inserted into the patient's body 52, the needle holder 67 is raised by the pinion gear 71 provided to the lower case 57. This avoids the generation of a snapping sound made by a spring during a conventional needle extraction operation, which reduces patient discomfort (fear).

Furthermore, with the sensor insertion device 51 in this embodiment, the pinion gear 71, the first rack gear 72, and the second rack gear 73 constituting the needle holder raising mechanism 74 are disposed near the center axis of the cylindrical lower case 57 and near the center axis of the needle holder 67, as shown in FIGS. 26 and 31. Therefore, the needle holder 67 receives the lifting force of the needle holder raising mechanism 74 near its central axis, so the needle holder 67 can be raised stably and with good balance.

As a result, a stable needle extraction operation can be performed.

With the sensor insertion device 51 in this embodiment, the lower end side of the second rack gear 73 fixed to the upper case 58 is formed as a flexible free end, as shown in FIG. 31. Accordingly, when the second rack gear 73 descends and comes into contact with the pinion gear 71, the distal end side of the second rack gear 73 bends in the direction away from the pinion gear 71, so the second rack gear 73 can be properly engaged with the pinion gear 71.

As a result, a stable needle extraction operation can be performed.

Furthermore, with the sensor insertion device 51 in this embodiment, as shown in FIG. 26, the needle holder engaging portions 79 are provided at three places on the upper surface of the needle holder 67. The first rack gear 72 is provided between two adjacent needle holder engaging portions 79.

Accordingly, during the needle insertion operation, since the first rack gear 72 is provided between two adjacent needle holder engaging portions 79, the first rack gear 72 can be lowered stably. Therefore, the first rack gear 72 that has been lowered in a stable state rotates the pinion gear 71, so a stable needle insertion operation can be performed.

Furthermore, with the sensor insertion device 51 in this embodiment, as shown in FIG. 31, the needle unit 77 holding the guide needle 78 is fixed between two adjacent needle holder engaging portions 79 on the lower surface of the needle holder 67. More specifically, the guide needle 78 is provided directly below the first rack gear 72.

Therefore, during the needle insertion operation, the needle unit 77 also descends in a stable state directly below the first rack gear 72 that is lowered in a stable state. As a result, a stable needle insertion operation can be performed.

Furthermore, in the sensor insertion device 51 in this embodiment, the needle holder engaging portions 79 of the needle holder 67 are provided at three places at equal angular intervals along the outer peripheral portion of the needle holder 67. That is, the needle holder engaging portions 79 are disposed so as to form an equilateral triangle.

Therefore, since the three needle holder engaging portions 79 press the needle holder 67 down at equal angular intervals, a more stable needle insertion operation can be performed.

Furthermore, with the sensor insertion device 51 in this embodiment, as shown in FIG. 26, the upper case 58 has a cylindrical shape with an open lower surface, and the contact portion 64 that comes into contact with the palm of the user is formed on the upper surface portion 63 of the upper case 58.

Therefore, the user can press down the upper case 58 with the palm, with which it is easy to apply force, in the needle insertion operation and the needle extraction operation, and thus can perform stable needle insertion and needle extraction.

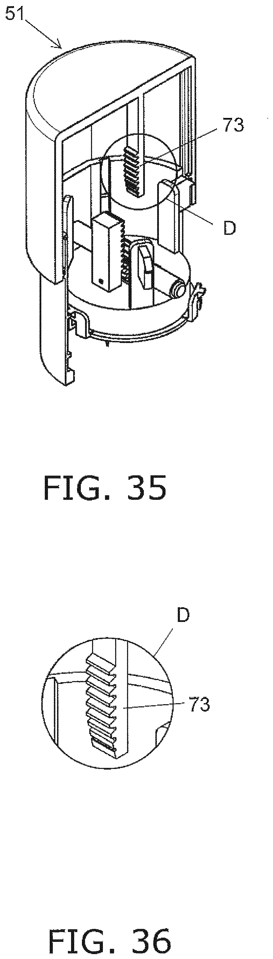

The second rack gear 73 here may be configured such that its lower end side is inclined to the opposite side from the pinion gear 71, that is, to the side farther away from the pinion gear 71, as shown in the portion D in FIG. 35 and in FIG. 36.

That is, the second rack gear 73 is inclined so as to move away from the pinion gear 71 as it proceeds downward.

With this configuration, since the lower end side of the second rack gear 73 is inclined away from the pinion gear 71, the lower end side of the second rack gear 73 can be gradually engaged with the pinion gear 71.

As a result, proper engagement can be achieved between the second rack gear 73 and the pinion gear 71, so a stable needle extraction operation can be performed.

The height of the engaging teeth formed on the lower end side of the second rack gear 73 may be lower than the height of the engaging teeth formed above the second rack gear 73.

With this configuration, since the lower end side of the second rack gear 73 is engaged with the pinion gear 71 from engaging teeth that are lower in height, the lower end side of the second rack gear 73 can be gradually engaged with the pinion gear 71.

As a result, just as described above, since proper engagement can be achieved between the second rack gear 73 and the pinion gear 71, a stable needle extraction operation can be performed.

As described above, the sensor insertion device 51 in this embodiment comprises the lower case 57, the upper case 58, the sensor base 54, the needle holder 67, and the needle holder raising mechanism 74. The lower case 57 has the upper surface opening 59. The upper case 58 is placed over the outer periphery of the lower case 57 from above, is disposed slidably with respect to the lower case, and is pressed downward by the user when a needle insertion operation is performed. The sensor base 54 is disposed in the lower case 57, and holds the sensor 56 for acquiring biological information. The needle holder 67 is disposed movably in the up and down direction above the sensor base 54, and holds the guide needle 78 that is inserted into the patient's body to guide the sensor 56 into the body. The needle holder raising mechanism 74 raises the needle holder 67 that holds the guide needle that has been inserted into the patient's body. The needle holder raising mechanism 74 has the pinion gear 71, the first rack gear 72, and the second rack gear 73. The pinion gear 71 raises the needle holder 67. The first rack gear 72 is provided to the needle holder 67 and is engaged with the pinion gear 71. The second rack gear 73 is provided to the upper case 58 and is engaged with the pinion gear 71. As the upper case 58 descends, the pinion gear 71 raises the needle holder 67 by engaging with the second rack gear 73.

That is, with the sensor insertion device 51 in this embodiment, after the guide needle 78 of the needle holder 67 has been inserted, the upper case 58 is pressed further downward, so that the needle holder 67 is raised by the pinion gear 71 and the first and second rack gears 72 and 73 included in the needle holder raising mechanism 74. Therefore, no snapping sound is produced by a spring, and patient discomfort (fear) can be reduced.

### INDUSTRIAL APPLICABILITY

The present invention is anticipated to find use as a sensor insertion device for inserting a sensor for measuring biological information into a patient's body, for performing continuous blood glucose measurement, for example.

### REFERENCE SIGNS LIST

1 sensor insertion device
2 patient's body
3 sensor unit
4 sensor base
5 measuring device (biological information measuring device)
6 sensor
7 lower case
8 upper case
9 upper surface opening
10 bottom outlet (lower opening of antibacterial chamber)
11 lower surface opening
12 long side surface
13 grip portion
14 slide guide
15 long side surface
16 upper opening
17 antibacterial wall
18 sensor holder
19 needle unit
20 guide needle
21 through-hole
22 antibacterial sheet (first antibacterial sheet)
23 bottom
24 antibacterial sheet (second antibacterial sheet)
25 antibacterial chamber
26 needle holder
27 engaging arm
28 engagement hole
29 short side surface
30 pinion gear
30A needle holder raising mechanism
31 shaft
32 shaft groove
33 rack gear (first rack gear)
34 rack gear (second rack gear)
35 shaft support
36 needle carrier
37 engagement protrusion
38 concave portion
39 blade
40 conical portion
41 antibacterial sheet holder
42 O-ring
43 adhesive portion
44 holding prong
51 sensor insertion device
52 patient's body
53 sensor unit
54 sensor base
55 measuring device
56 sensor
57 lower case
58 upper case
59 upper surface opening
60 bottom outlet
61 lower surface opening
62 grip portion
63 upper surface
64 contact portion
65 slide guide
66 base holder
67 needle holder
68 skin tape
69 base engaging portion
70 first engaging prong
71 pinion gear
72 first rack gear
73 second rack gear
74 needle holder raising mechanism
75 shaft
76 shaft support
77 needle unit
78 guide needle
79 needle holder engaging portion
80 needle holder pressing portion
81 lower case engaging portion
82 upper case engaging prong
83 second engaging prong
84 needle accommodation portion

## Claims

1. A sensor insertion device (1,51), comprising:
a lower case (7,57) that has an upper surface opening (9,59);
an upper case (8,58) that is disposed around an outer periphery of the lower case (7,57), covering the lower case from above, and configured to be slidable with respect to the lower case (7,57), and that is configured to be operated in direction of needle insertion by a user when performing a needle insertion operation;
a sensor base (4,54) that is disposed inside the lower case (7,57) and that is is configured to hold a sensor (6,56) configured to acquire biological information;
a needle holder (26,67) that is disposed above the sensor base (4,54) and configured to be movable up and down, and that is configured to hold a guide needle (20,78) which is configured to be inserted into a patient's body (2,52) in order to guide the sensor (6,56) into the patient's body (2,52); and
a needle holder raising mechanism (30A,74) configured to raise the needle holder (26,67) holding the guide needle (20,78) that has been inserted into the patient's body (2,52),
the needle holder raising mechanism (30A,74) has:
a pinion gear (30,71) configured to raise the needle holder (26,67);
a first rack gear (33,72) that is provided on the needle holder (26,67) and is engaged with the pinion gear (30,71); and
a second rack gear (34,73) that is provided on the upper case (8,58) and is engaged with the pinion gear (30,71), and
as the upper case (8,58) is further operated in direction of needle insertion, the pinion gear (30,71) is configured to raise the needle holder (26,67) by engagement with the second rack gear (34,73).

2. The sensor insertion device (1,51) according to claim 1,
wherein when the upper case (8,58) is lowered close to a position on the body that will be punctured by the guide needle (20,78), the pinion gear (30,71) is configured to engage with a lower end portion of the second rack gear (34,73), and when the upper case (8,58) is further lowered, the second rack gear (34,73) is configured to rotate the pinion gear (30,71) and cause the needle holder (26,67) to rise along with the first rack gear (33,72).

3. The sensor insertion device (1,51) according to claim 1 or 2,
wherein the second rack gear (34,73) is in a state of being disengaged from the pinion gear (30,71) prior to the needle insertion operation.

4. The sensor insertion device (1,51) according to any of claims 1 to 3,
wherein the second rack gear (34,73) has a fixed end that is fixed on an upper end side and is fixed to the upper case (8,58), and a free end that is provided on a lower end side and is flexible.

5. The sensor insertion device (1,51) according to any of claims 1 to 4,
wherein the lower end side of the second rack gear (34,73) is inclined away from the pinion gear (30,71).

6. The sensor insertion device (1,51) according to any of claims 1 to 5,
wherein the second rack gear (34,73) is such that a height of the engagement teeth formed on the lower end side is less than a height of the engagement teeth formed above the lower end side.

7. The sensor insertion device (1,51) according to any of claims 1 to 6,
wherein the first rack gear (33,72) is disposed facing upward from an upper surface of the needle holder (26,67).

8. The sensor insertion device (1,51) according to any of claims 1 to 7,
wherein the first rack gear (33,72) is engaged with the pinion gear (30,71) before and after the needle insertion operation.

9. The sensor insertion device (1,51) according to any of claims 1 to 8,
wherein the needle holder (26,67) has a needle holder engaging portion (79) in at least three places, and
the upper case (8,58) has on its inner surface a needle holder pressing part (80) that is provided on a portion opposite the needle holder engaging portion (79).

10. The sensor insertion device (1,51) according to claim 9,
wherein the first rack gear (33,72) is provided between two adjacent needle holder engaging portions (79).

11. The sensor insertion device (1,51) according to claim 9 or 10,
wherein the guide needle (20,78) is fixed between two adjacent needle holder engaging portions (79) on a lower surface of the needle holder (26,67).

12. The sensor insertion device (1,51) according to any of claims 9 to 11,
wherein the needle holder engaging portions (79) are provided at three places, equidistantly spaced apart.

13. The sensor insertion device (1,51) according to any of claims 1 to 12,
wherein the lower case (7,57) has on its inner surface a shaft support portion on which a shaft (31, 75) of the pinion gear (30,71) is supported.

14. The sensor insertion device (1,51) according to any of claims 1 to 13,
wherein the lower case (7,57) has a substantially cylindrical shape, and
the needle holder raising mechanism (30A,74) is disposed near a center of the lower case (7,57).

15. The sensor insertion device (1,51) according to any of claims 1 to 14,
wherein the upper case (8,58) has on its upper surface a contact portion (64) that comes into contact with a palm of the user.

## Patentansprüche

1. Sensoreinführvorrichtung (1, 51) mit:
einem unteren Gehäuse (7, 57), das eine Öffnung (9, 59) an einer oberen Fläche aufweist,
einem oberen Gehäuse (8, 58), das um eine äußere Peripherie des unteren Gehäuses (7, 57) angeordnet ist, wobei es das untere Gehäuse von oben abdeckt und ausgestaltet ist, hinsichtlich des unteren Gehäuses (7, 57) verschiebbar zu sein, und das ausgestaltet ist, in einer Richtung einer Nadeleinführung durch einen Benutzer bedient zu werden, wenn eine Nadeleinführbedienung durchgeführt wird,
einer Sensorbasis (4, 54), die innerhalb des unteren Gehäuses (7, 57) angeordnet ist und die ausgestaltet ist, einen Sensor (6, 56) zu halten, der ausgestaltet ist, biologische Information zu erhalten,
einem Nadelhalter (26, 67), der oberhalb der Sensorbasis (4, 54) angeordnet ist und ausgestaltet ist, aufwärts und abwärts beweglich zu sein, und der ausgestaltet ist, eine Führungsnadel (20, 78) zu halten, die ausgestaltet ist, in einen Patientenkörper (2, 52) eingeführt zu werden, um den Sensor (6, 56) in den Patientenkörper (2, 52) zu führen, und
einem Nadelhalteranhebemechanismus (30A, 74), der ausgestaltet ist, den Nadelhalter (26, 67), der die Führungsnadel (20, 78) hält, die in den Patientenkörper (2, 52) eingeführt wurde, anzuheben,
wobei der Nadelhalteranhebemechanismus (30A, 74) aufweist:
ein Ritzel (30, 71), das ausgestaltet ist, den Nadelhalter (26, 67) anzuheben,
eine erste Zahnstange (33, 72), die an dem Nadelhalter (26, 67) vorgesehen ist und mit dem Ritzel (30, 71) in Eingriff steht, und
eine zweite Zahnstange (34, 73), die an dem oberen Gehäuse (8, 58) vorgesehen ist und mit dem Ritzel (30, 71) in Eingriff steht, und
wenn das obere Gehäuse (8, 58) weiter in Richtung der Nadeleinführung bedient wird, das Ritzel (30, 71) ausgestaltet ist, den Nadelhalter (26, 67) durch Eingriff mit der zweiten Zahnstange (34, 73) anzuheben.

2. Sensoreinführvorrichtung (1, 51) nach Anspruch 1,
wobei, wenn das obere Gehäuse (8, 58) in die Nähe einer Position an dem Körper heruntergelassen wird, der durch die Führungsnadel (20, 78) punktiert wird, das Ritzel (30, 71) ausgestaltet ist, mit einem unteren Endabschnitt der zweiten Zahnstange (34, 73) in Eingriff zu kommen, und wenn das obere Gehäuse (8, 58) weiter heruntergedrückt wird, die zweite Zahnstange (34, 73) ausgestaltet ist, das Ritzel (30, 71) zu rotieren und zu veranlassen, dass der Nadelhalter (26, 67) zusammen mit der ersten Zahnstange (33, 72) angehoben wird.

3. Sensoreinführvorrichtung (1, 51) nach Anspruch 1 oder 2,
wobei die zweite Zahnstange (34, 73) vor der Nadeleinführbedienung in einem Zustand ist, bei dem sie mit dem Ritzel (30, 71) nicht im Eingriff steht.

4. Sensoreinführvorrichtung (1, 51) nach einem der Ansprüche 1 bis 3,
wobei die zweite Zahnstange (34, 73) ein fixiertes Ende aufweist, das an einer oberen Endseite fixiert ist und an dem oberen Gehäuse (8, 58) fixiert ist, und ein freies Ende aufweist, das an einer unteren Endseite vorgesehen ist und flexibel ist.

5. Sensoreinführvorrichtung (1, 51) nach einem der Ansprüche 1 bis 4,
wobei die untere Endseite der zweiten Zahnstange (34, 73) von dem Ritzel (30, 71) weggeneigt ist.

6. Sensoreinführvorrichtung (1, 51) nach einem der Ansprüche 1 bis 5,
wobei die zweite Zahnstange (34, 73) derart ausgestaltet ist, dass eine Höhe der Eingriffszähne, die an der unteren Endseite gebildet sind, geringer ist als eine Höhe der Eingriffszähne, die oberhalb der unteren Endseite gebildet sind.

7. Sensoreinführvorrichtung (1, 51) nach einem der Ansprüche 1 bis 6,
wobei die erste Zahnstange (33, 72) angeordnet ist, so dass sie von einer oberen Fläche des Nadelhalters (26, 73) aufwärts weist.

8. Sensoreinführvorrichtung (1, 51) nach einem der Ansprüche 1 bis 7,
wobei die erste Zahnstange (33, 72) mit dem Ritzel vor und nach der Nadeleinführbedienung im Eingriff steht.

9. Sensoreinführvorrichtung (1, 51) nach einem der Ansprüche 1 bis 8,
wobei der Nadelhalter (26, 67) einen Nadelhaltereingriffsabschnitt (79) an wenigstens drei Stellen aufweist, und
das obere Gehäuse (8, 58) an seiner inneren Fläche einen Nadelhalterdruckteil (80) aufweist, der an einem Abschnitt gegenüberliegend des Nadelhaltereingriffsabschnitts (79) vorgesehen ist.

10. Sensoreinführvorrichtung (1, 51) nach Anspruch 9,
wobei die erste Zahnstange (33, 72) zwischen zwei benachbarten Nadelhaltereingriffsabschnitten (79) vorgesehen ist.

11. Sensoreinführvorrichtung (1, 51) nach Anspruch 9 oder 10,
wobei die Führungsnadel (20, 78) zwischen zwei benachbarten Nadelhaltereingriffsabschnitten (79) an einer unteren Fläche des Nadelhalters (26, 67) fixiert ist.

12. Sensoreinführvorrichtung (1, 51) nach einem der Ansprüche 9 bis 11,
wobei die Nadelhaltereingriffsabschnitte (79) an drei Stellen vorgesehen sind, die in gleichem Abstand vorgesehen sind.

13. Sensoreinführvorrichtung (1, 51) nach einem der Ansprüche 1 bis 12,
wobei das untere Gehäuse (7, 57) an seiner inneren Fläche einen Wellenstützabschnitt aufweist, an dem eine Welle (31, 75) des Ritzels (30, 71) gestützt ist.

14. Sensoreinführvorrichtung (1, 51) nach einem der Ansprüche 1 bis 13,
wobei das untere Gehäuse (7, 57) im Wesentlichen zylindrische Form aufweist und
der Nadelhalteranhebemechanismus (30A, 74) in der Nähe einer Mitte des unteren Gehäuses (7, 57) angeordnet ist.

15. Sensoreinführvorrichtung (1, 51) nach einem der Ansprüche 1 bis 14,
wobei das obere Gehäuse (8, 58) an seiner oberen Fläche einen Kontaktabschnitt (64) aufweist, der mit einer Handfläche des Benutzers in Kontakt kommt.

## Revendications

1. Dispositif d'insertion de capteur (1, 51) comprenant:
un boîtier inférieur (7, 57) qui présente une ouverture de surface supérieure (9, 59),
un boîtier supérieur (8, 58) qui est disposé autour d'une périphérie externe du boîtier inférieur (7, 57), dans lequel il recouvre depuis le dessus le boîtier inférieur et est configuré de manière à pouvoir coulisser par rapport au boîtier inférieur (7, 57), et qui est configuré de manière à être actionné dans une direction d'une insertion de l'aiguille par un utilisateur lors de l'exécution d'une opération d'insertion de l'aiguille;
une base de capteur (4, 54) qui est disposée à l'intérieur du boîtier inférieur (7, 57) et qui est configurée pour maintenir un capteur (6, 56) qui est configuré pour acquérir de l'information biologique,
un porte-aiguille (26, 67) qui est disposé au-dessus de la base de capteur (4, 54) et est configuré de manière à être mobile vers le haut et vers le bas, et qui est configuré pour maintenir une aiguille de guidage (20, 78) qui est configurée pour être insérée dans le corps d'un patient (2, 52) afin de guider le capteur (6, 56) dans le corps du patient (2, 52), et
un mécanisme de levage de porte-aiguille (30A, 74) qui est configuré pour lever le porte-aiguille (26, 67) maintenant l'aiguille de guidage (20, 78) qui a été insérée dans le corps du patient (2, 52),
dans lequel le mécanisme de levage de porte-aiguille (30A, 74) comprend:
un pignon (30, 71) qui est configuré pour lever le porte-aiguille (26, 67),
une première crémaillère (33, 72) qui est prévue sur le porte-aiguille (26,67) et est en prise avec le pignon (30,71), et
une deuxième crémaillère (34, 73) qui est prévue sur le boîtier supérieur (8, 58) et est en prise avec le pignon (30, 71), et
lorsque le boîtier supérieur (8, 58) est en outre actionné dans la direction de l'insertion de l'aiguille, le pignon (30, 71) est configuré pour lever le porte-aiguille (26, 67) par engagement avec la deuxième crémaillère (34, 73).

2. Dispositif d'insertion de capteur (1, 51) selon la revendication 1,
dans lequel lorsque le boîtier supérieur (8, 58) est abaissé à proximité d'une position sur le corps qui sera ponctionné par l'aiguille de guidage (20, 78), le pignon (30, 71) est configuré pour venir en prise avec une portion d'extrémité inférieure de la deuxième crémaillère (34, 73), et lorsque le boîtier supérieur (8,58) est encore abaissé, la deuxième crémaillère (34, 73) est configurée pour faire tourner le pignon (30, 71) et pour provoquer que le porte-aiguille (26, 67) est levé conjointement avec la première crémaillère (33, 72).

3. Dispositif d'insertion de capteur (1, 51) selon la revendication 1 ou 2,
dans lequel, avant l'opération d'insertion de l'aiguille, la deuxième crémaillère (34, 73) est dans un état où elle est désengagée du pignon (30, 71).

4. Dispositif d'insertion de capteur (1, 51) selon l'une quelconque des revendications 1 à 3,
dans lequel la deuxième crémaillère (34, 73) présente une extrémité fixée qui est fixée sur un côté d'extrémité supérieure et est fixée au boîtier supérieur (8, 58), ainsi qu'une extrémité libre qui est prévue sur un côté d'extrémité inférieure et est flexible.

5. Dispositif d'insertion de capteur (1, 51) selon l'une quelconque des revendications 1 à 4,
dans lequel le côté d'extrémité inférieure de la deuxième crémaillère (34, 73) est incliné en s'éloignant du pignon (30, 71).

6. Dispositif d'insertion de capteur (1, 51) selon l'une quelconque des revendications 1 à 5,
dans lequel la deuxième crémaillère (34, 73) est telle qu'une hauteur des dents d'engagement formées sur le côté extrémité inférieure est inférieure à une hauteur des dents d'engagement formées au-dessus du côté d'extrémité inférieure.

7. Dispositif d'insertion de capteur (1, 51) selon l'une quelconque des revendications 1 à 6,
dans lequel la première crémaillère (33, 72) est disposée de manière à montrer vers le haut depuis une surface supérieure du porte-aiguille (26, 67).

8. Dispositif d'insertion de capteur (1, 51) selon l'une quelconque des revendications 1 à 7,
dans lequel la première crémaillère (33, 72) est en prise avec le pignon (30, 71) avant et après l'opération d'insertion de l'aiguille.

9. Dispositif d'insertion de capteur (1, 51) selon l'une quelconque des revendications 1 à 8,
dans lequel le porte-aiguille (26, 67) comprend une portion d'engagement de porte-aiguille (79) à au moins trois endroits, et
le boîtier supérieur (8, 58) comprend sur sa surface intérieure une partie de pression de porte-aiguille (80) qui est prévue sur une portion opposée à la portion d'engagement de porte-aiguille (79).

10. Dispositif d'insertion de capteur (1, 51) selon la revendication 9,
dans lequel la première crémaillère (33, 72) est prévue entre deux portions adjacentes d'engagement de porte-aiguille (79).

11. Dispositif d'insertion de capteur (1, 51) selon la revendication 9 ou 10,
dans lequel l'aiguille de guidage (20, 78) est fixée entre deux portions adjacentes d'engagement de porte-aiguille (79) sur une surface inférieure du porte-aiguille (26, 67).

12. Dispositif d'insertion de capteur (1, 51) selon l'une quelconque des revendications 9 à 11,
dans lequel les portions d'engagement de porte-aiguille (79) sont prévues à trois endroits prévus à distance égale les uns des autres.

13. Dispositif d'insertion de capteur (1, 51) selon l'une quelconque des revendications 1 à 12,
dans lequel le boîtier inférieur (7, 57) comprend sur sa surface intérieure une portion de support d'arbre sur laquelle est supporté un arbre (31, 75) du pignon (30, 71).

14. Dispositif d'insertion de capteur (1, 51) selon l'une quelconque des revendications 1 à 13,
dans lequel le boîtier inférieur (7, 57) présente une forme pour l'essentiel cylindrique, et
le mécanisme de levage de porte-aiguille (30A, 74) est disposé à proximité d'un centre du boîtier inférieur (7, 57).

15. Dispositif d'insertion de capteur (1, 51) selon l'une quelconque des revendications 1 à 14,
dans lequel le boîtier supérieur (8, 58) présente sur sa surface supérieure une portion de contact (64) qui entre en contact avec une paume de l'utilisateur.
